# FASCICULE DE BREVET EUROPEEN

(11) **EP 3 852 640 B1**
(45) Date de publication et mention de la délivrance du brevet: **20.03.2024**
(21) Numéro de dépôt: 19768825.2
(22) Date de dépôt: 16.09.2019
(51) Int. Cl.: A61B 8/08, A61B 8/00

(54) **SONDE D'ELASTOGRAPHIE TRANSITOIRE AVEC MEMBRANE D'ETANCHEITE INTEGREE AU TRANSDUCTEUR ULTRASONORE**
SONDE FÜR TRANSIENTE ELASTOGRAFIE MIT IN DEN ULTRASCHALLWANDLER INTEGRIERTER ABDICHTUNGSMEMBRAN
PROBE FOR TRANSIENT ELASTOGRAPHY WITH SEALING MEMBRANE INTEGRATED INTO THE ULTRASOUND TRANSDUCER

(30) Priorité: 18.09.2018 FR 1858409
(43) Date de publication de la demande: 28.07.2021
(73) Titulaire: Echosens, 75014 Paris (FR)
(72) Inventeur: SANDRIN, Laurent, 92340 BOURG-LA-REINE (FR)
(74) Mandataire: Cabinet Camus Lebkiri
(86) Numéro de dépôt international: PCT/EP2019/074700
(87) Numéro de publication internationale: WO 2020/058188

(56) Documents cités:
- FR-A1- 2 843 290
- US-A1- 2018 140 274

## Description

### DOMAINE TECHNIQUE DE L'INVENTION

Le domaine technique de l'invention est celui des sondes d'élastographie transitoire et plus particulièrement celui des sondes d'élastographie transitoire comportant un transducteur ultrasonore intégrant une membrane d'étanchéité.

La présente invention concerne une sonde d'élastographie transitoire qui peut être utilisée pour la mesure de propriétés viscoélastiques d'un tissu humain ou animal et en particulier une sonde d'élastographie transitoire comportant un transducteur ultrasonore pourvue d'une membrane d'étanchéité.

### ARRIERE-PLAN TECHNOLOGIQUE DE L'INVENTION

Plusieurs affections hépatiques peuvent être diagnostiquées en évaluant les propriétés viscoélastiques du tissu hépatique. Les hépatites virales chroniques, les stéato-hépatites d'origines alcooliques et non alcooliques, les hépatites auto-immunes, les hépatites virales, la cirrhose biliaire primaire sont toutes responsables d'un changement progressif de la rigidité du foie. Dans certains cas, cette augmentation de la rigidité, aussi appelée fibrose, peut mener à une cirrhose et à une insuffisance hépatique avec de graves conséquences pour le patient.

L'une des techniques les plus fiables et les plus efficaces pour mesurer la rigidité du foie est l'élastographie transitoire (voir par exemple "WFUMB guidelines and recommendations for clinical use of ultrasound elastography part 3 : liver" par G. Ferraioli et al. publié dans " Ultrasound in Med. And Biol.", 41, 5, 2015).

Le demandeur a mis au point et commercialisé un dispositif appelé Fibroscan^{®} (voir par exemple les brevets EP1169636 et EP1531733) qui mesure la rigidité du foie en utilisant une technique d'élastographie appelée "Vibration Controlled Transient Elastography" (VCTE) développée par le demandeur.

Dans une application VCTE, la mesure de la rigidité du foie repose sur la mesure d'une vitesse de propagation d'ondes de cisaillement transitoires à l'intérieur du tissu étudié. Pour effectuer une telle mesure, une sonde particulière a été mise au point. Cette sonde comprend au moins un vibreur et au moins un transducteur ultrasonore.

Par exemple, dans la sonde Fibroscan^{®}, le vibreur déplace le transducteur ultrasonore et le pousse contre le corps du patient. Ce mouvement pulsé génère une onde de cisaillement transitoire qui se propage à l'intérieur du foie. Le déplacement engendré par la propagation de l'onde de cisaillement est alors sondé par l'envoi de courtes impulsions ultrasonores à haute fréquence dans le milieu étudié.

Durant l'examen, le transducteur ultrasonore est alors soit directement en contact avec le corps du patient soit en contact avec un gel à base d'eau favorisant la transmission ultrasonique. Il est donc nécessaire que le transducteur ultrasonore soit résistant à l'eau pour empêcher sa détérioration, tout en permettant un examen efficace et non douloureux pour le patient.

Le document FR2843290A1 divulgue une sonde d'élastographie transitoire comportant un un corps de sonde, transducteur ultrasonore comprenant une face étant destinée à être en contacte avec le corps d'un patient, et un vibreur, le transducteur étant monté sur le vibreur et la sonde comportant une membrane d'étanchéité épousant les contours extérieurs du transducteur.

### RESUME DE L'INVENTION

L'invention offre une solution aux problèmes évoqués précédemment, en permettant de réaliser un examen d'élastographie transitoire indolore pour le patient et sans risque de détérioration de la sonde utilisée.

Un aspect de l'invention concerne une sonde d'élastographie transitoire comportant :
- Un corps de sonde ;
- Un transducteur ultrasonore configuré pour générer un faisceau ultrasonore selon un axe, le faisceau ultrasonore étant généré depuis une face du transducteur ultrasonore ;
- Un vibreur situé à l'intérieur du corps de sonde et agencé de manière à induire un mouvement du transducteur ultrasonore selon un axe prédéfini ;
le transducteur ultrasonore étant monté sur le vibreur de manière que l'axe prédéfini et l'axe du faisceau ultrasonore soient confondus, la sonde (100) comportant en outre une membrane d'étanchéité épousant les contours extérieurs du transducteur ultrasonore recouvrant la face du transducteur ultrasonore.

Grâce à l'invention, le transducteur ultrasonore est entouré d'une membrane permettant de rendre la sonde résistante à l'eau et donc d'éviter son endommagement pendant l'examen. La membrane permet également de recouvrir les points anguleux situés au niveau de la face du transducteur ultrasonore pour rendre l'examen indolore pour le patient. Enfin, la membrane simplifie l'obtention d'une isolation diélectrique suffisante pour la mise en conformité de la sonde vis-à-vis des réglementations en vigueur.

Outre les caractéristiques qui viennent d'être évoquées dans le paragraphe précédent, la sonde selon un aspect de l'invention peut présenter une ou plusieurs caractéristiques complémentaires parmi les suivantes, considérées individuellement ou selon toutes les combinaisons techniquement possibles. Avantageusement, l'ensemble transducteur ultrasonore et membrane constitue un embout détachable.

Ainsi, le transducteur ultrasonore utilisé peut être adapté au patient, le diamètre de la partie de la sonde en contact avec le patient étant différent selon la morphologie du patient.

Avantageusement, le transducteur ultrasonore présente un axe de symétrie correspondant à l'axe du faisceau ultrasonore.

Avantageusement, le transducteur ultrasonore est relié au corps de sonde par la membrane.

Avantageusement, la membrane est en élastomère.

Ainsi, la membrane est flexible et se déforme lorsque le transducteur ultrasonore bouge.

Avantageusement, la membrane est en élastomère de type silicone.

Avantageusement, la partie de la membrane en contact avec la face du transducteur ultrasonore forme une lentille acoustique configurée pour focaliser le faisceau ultrasonore.

Ainsi, le faisceau ultrasonore est mieux focalisé, ce qui permet d'obtenir des mesures plus précises. Dans ce cas, la partie de la membrane située devant la face du transducteur ultrasonore sera convexe ou concave selon que la vitesse de propagation des ultrasons dans la lentille est inférieure ou supérieure à celle des ultrasons dans l'eau.

Avantageusement, la partie de la membrane en contact avec la face du transducteur ultrasonore est convexe.

Avantageusement, la partie de la membrane en contact avec la face du transducteur ultrasonore est concave.

Avantageusement, la membrane est réalisée dans un matériau électriquement isolant.

Ainsi, la membrane permet une meilleure isolation diélectrique de la sonde. En effet, dans la configuration actuelle, il est plus difficile de réaliser l'isolation diélectrique car le transducteur ultrasonore est muni d'une membrane qui ne recouvre que la face du transducteur ultrasonore. Aussi des fuites diélectriques peuvent intervenir sur le périmètre de la membrane.

Avantageusement, la membrane est collée au transducteur ultrasonore.

Avantageusement, la partie de la membrane située entre le transducteur ultrasonore et le corps de sonde est déformable.

Ainsi, la membrane se déforme lorsque le transducteur ultrasonore se déplace sous l'effet du vibreur situé dans le corps de sonde.

Avantageusement, le diamètre extérieur de la partie de la membrane en contact avec la face du transducteur ultrasonore est compris entre 3 et 25 mm.

Avantageusement, l'épaisseur de la partie de la membrane en contact avec la face du transducteur ultrasonore est comprise entre 50 µm et 5 mm.

Avantageusement, tout ou partie du transducteur ultrasonore a une forme de cône tronqué, la face du transducteur ultrasonore correspondant à la base du cône tronqué d'aire minimale.

Ainsi, la surface en contact avec le patient est moins large ce qui permet de loger plus facilement la face du transducteur ultrasonore dans l'espace intercostal du patient tout en ménageant de la place pour l'aménagement de composants électroniques au niveau d'une face arrière plus large.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1A montre une représentation schématique de la sonde d'élastographie transitoire selon un premier aspect de l'invention.
- La figure 1B montre une représentation schématique de la sonde d'élastographie transitoire selon un premier aspect de l'invention de laquelle a été détachée un embout constitué par le transducteur ultrasonore recouvert de la membrane d'étanchéité.
- La figure 2A montre une représentation schématique d'un transducteur ultrasonore de forme conique et une membrane de forme circulaire.
- La figure 2B montre une représentation schématique du transducteur ultrasonore représentée à la figure 2A recouvert de la membrane représentée à la figure 2A qui constitue un embout détachable.
- La figure 3A montre une représentation schématique des ondes ultrasonores émises par le transducteur ultrasonore en l'absence de membrane et plus particulièrement les ondes ultrasonores réfléchies sur un point F situé à une distance d du transducteur ultrasonore.
- La figure 3B montre une représentation schématique des ondes ultrasonores émises par le transducteur ultrasonore recouvert de la membrane constituant une lentille acoustique et plus particulièrement les ondes ultrasonores réfléchies sur un point F situé à une distance d du transducteur ultrasonore et constituant le foyer de la lentille acoustique.
- La figure 3C montre la réponse impulsionnelle de diffraction au point F des figures 3A et 3B quand le transducteur ultrasonore est dépourvu de la membrane et quand le transducteur ultrasonore est pourvu de la membrane.

### DESCRIPTION DETAILLEE D'AU MOINS UN MODE DE REALISATION DE

### L'INVENTION

Sauf précision contraire, un même élément apparaissant sur des figures différentes présente une référence unique.

Un premier aspect de l'invention concerne une sonde d'élastographie transitoire. On entend par « sonde d'élastographie transitoire » une sonde permettant la mise en oeuvre de la technologie VCTE pour « Vibration Controlled Transient Elastography », c'est-à-dire une sonde permettant d'estimer la vitesse de propagation d'une onde de cisaillement basse fréquence dans le milieu étudié en utilisant des ondes ultrasonores ultra haute fréquence pour mesurer les déplacements locaux du milieu lors du passage de l'onde de cisaillement. La vitesse de propagation permet alors d'estimer un paramètre viscoélastique du milieu.

Dans la suite de la description, on emploiera indifféremment les termes « sonde », et « sonde d'élastographie transitoire ».

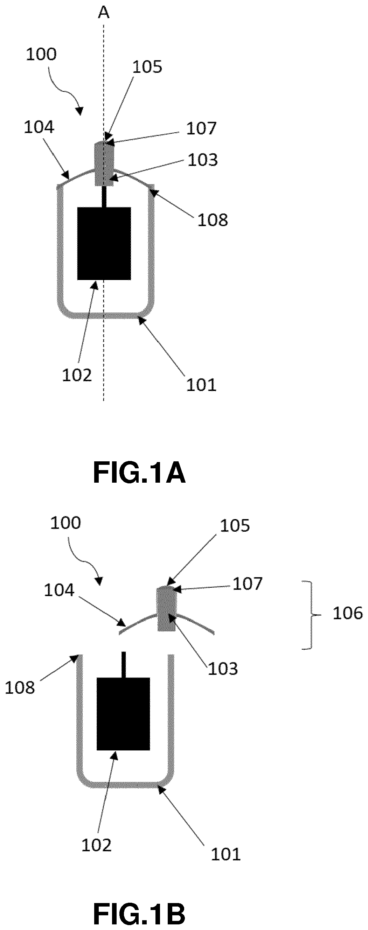

La sonde 100 selon un premier aspect de l'invention est représentée à la figure 1A.

La sonde 100 comporte :
- un corps de sonde 101 ;
- un vibreur 102 ;
- un transducteur ultrasonore 103 ; et
- une membrane 104.

Le transducteur ultrasonore 103 est configuré pour générer un faisceau ultrasonore. Le faisceau ultrasonore est généré au niveau d'une face 107 du transducteur ultrasonore 103.

Par exemple, le transducteur ultrasonore 103 présente un axe A de symétrie. L'axe de propagation du faisceau ultrasonore est alors parallèle à l'axe A de symétrie.

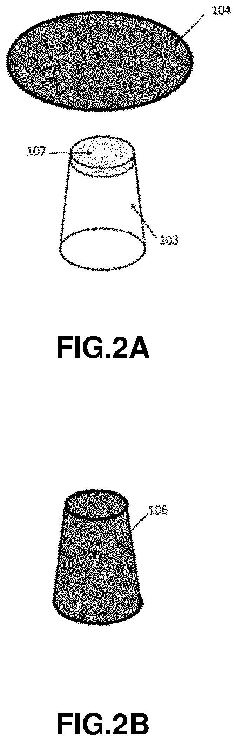

Par exemple, le transducteur ultrasonore 103 a une forme de cône tronqué comme représenté à la figure 2A ou une forme cylindrique et l'axe du cône ou l'axe du cylindre est alors l'axe A de symétrie.

Le transducteur ultrasonore a par exemple une longueur supérieure à 10 mm.

Sur la figure 2A, la face 107 correspond à la petite base ou base d'aire minimale du transducteur ultrasonore 103 de forme conique. En effet, un cône tronqué possède deux bases situées dans des plans parallèles, appelées petite base et grande base, la petite base ayant une aire inférieure à la grande base.

Sur la figure 1A, la face 107 correspond à la base du transducteur ultrasonore 103 de forme cylindrique qui n'est pas en contact avec le vibreur 102.

Le corps de sonde 101 a une forme permettant à la sonde 100 d'être tenue dans la main d'un opérateur durant l'examen d'élastographie transitoire. Par exemple, le corps de sonde 101 a la forme d'un solide de révolution ayant un axe confondu avec l'axe A de symétrie du transducteur ultrasonore 103, plus particulièrement le corps de sonde 101 a une forme cylindrique, l'axe A de symétrie du transducteur ultrasonore 103 étant l'axe du cylindre. Le corps de sonde 101 comporte alors une extrémité 108 extérieure de forme circulaire.

Les dimensions du corps de sonde 101 sont choisies pour permettre à un opérateur de tenir la sonde 100 dans sa main. Par exemple, dans le cas où le corps de sonde 101 est de forme cylindrique, le diamètre extérieur du corps de sonde 101 est compris entre 20 et 80 mm.

Le vibreur 102 est situé à l'intérieur du corps de sonde 101 et le transducteur ultrasonore 103 est monté sur le vibreur 102.

Le vibreur 102 est configuré pour induire un mouvement du transducteur ultrasonore 103 selon un axe prédéfini qui est confondu avec l'axe A de symétrie du transducteur ultrasonore 103. Ce mouvement permet de pousser le transducteur ultrasonore 103 contre le corps du patient pendant l'examen d'élastographie transitoire et génère ainsi une onde de cisaillement basse fréquence.

Le transducteur ultrasonore 103 est en contact avec le corps du patient au niveau de sa face 107.

Le fait que le transducteur ultrasonore 103 de forme conique soit en contact avec le patient au niveau de la petite base du cône tronqué, comme représenté à la figure 2A, fait que la face 107 a une surface plus petite qu'une face arrière correspondant à la grande base du cône tronqué, ce qui facilite le placement de la sonde 100 dans l'espace intercostal du patient tout en laissant suffisamment de place pour loger des composants électroniques au niveau de la face arrière.

Le transducteur ultrasonore 103 est recouvert d'une membrane 104 qui épouse ses contours extérieurs et assure l'étanchéité du transducteur ultrasonore 103. Par exemple, la membrane 104 est collée au transducteur ultrasonore 103. La membrane 104 peut également être surmoulée sur le transducteur ultrasonore 103.

Le fait que la membrane 104 recouvre l'intégralité du transducteur ultrasonore 103 permet d'éviter les fuites diélectriques qui interviennent lorsque la membrane 104 ne recouvre que la face 107 du transducteur ultrasonore 103.

Une partie 105 de la membrane 104 recouvre la face 107 du transducteur ultrasonore 103.

La membrane 104 permet alors de recouvrir les points anguleux au niveau de la face 107 du transducteur ultrasonore 103 pour rendre l'examen indolore pour le patient.

Par exemple, la partie de la membrane 104 située entre le corps de sonde 101 et le transducteur ultrasonore 103 et en particulier la partie de la membrane 104 destinée à être en contact avec le patient, est déformable. Ainsi, la membrane 104 se déforme lorsque le transducteur ultrasonore 103 se déplace sous l'effet du vibreur 102 situé dans le corps de sonde 101.

Par exemple, la membrane 104 est réalisée dans un matériau élastomère, conférant à la membrane 104 des propriétés élastiques. Plus particulièrement, la membrane 104 est réalisée en silicone.

Par exemple, la membrane 104 est réalisée dans un matériau isolant pour assurer une meilleure isolation diélectrique de la sonde 100.

Selon un mode de réalisation représenté à la figure 1A, le transducteur ultrasonore 103 est relié au corps de sonde 101 par le biais de la membrane 104.

Par exemple, la membrane 104 a une forme circulaire et les contours de la membrane 104 sont agencés sur le périmètre de l'extrémité 108 du corps de sonde 101. L'extrémité 108 du corps de sonde 101 comporte par exemple une rainure permettant d'insérer les contours de la membrane 104. La membrane 104 peut encore être par exemple collée ou clipsée au corps de sonde 101.

Ainsi, l'ensemble transducteur ultrasonore 103 et membrane 104 est facilement interchangeable et constitue un embout détachable 106 comme illustré à la figure 1B ou à la figure 2B.

Il est alors possible d'utiliser des embouts 106 de différentes tailles pour adapter les propriétés des ondes ultrasonores émises à la morphologie du patient. Par exemple, l'embout 106 peut comporter un transducteur ultrasonore 103 choisis parmi les transducteurs ultrasonores suivants :
- un transducteur ultrasonore 103 de fréquence centrale 8 MHz et de diamètre 3 mm ;
- un transducteur ultrasonore 103 de fréquence centrale 5 MHz et de diamètre 5 mm, l'embout 106 équipé de ce type de transducteur ultrasonore pouvant servir à mesurer l'élasticité du foie d'enfants ou d'adultes de petite taille ;
- un transducteur ultrasonore 103 de fréquence centrale 3.5 MHz et de diamètre 7 mm, l'embout 106 équipé de ce type de transducteur ultrasonore pouvant servir à mesurer l'élasticité du foie d'adultes ;
- un transducteur ultrasonore 103 de fréquence centrale 2.5 MHz et de diamètre 10 mm, l'embout 106 équipé de ce type de transducteur ultrasonore pouvant servir à mesurer l'élasticité du foie d'adultes obèses ;
- un transducteur ultrasonore 103 de fréquence centrale 1.5 MHz et de diamètre 12 mm.

En effet, plus le diamètre du transducteur ultrasonore 103 est petit et moins les ondes ultrasonores émises par le transducteur ultrasonore 103 parcourent de distance dans le milieu. Ainsi, dans le cas d'un patient obèse, la couche de tissu adipeux entre la peau et le foie est plus importante que pour un patient non-obèse et le diamètre du transducteur ultrasonore 103 doit donc être plus important pour que les mesures soient réalisées dans le foie et non dans le tissu adipeux.

L'embout 106 peut être par exemple vissé ou clipsé au corps de sonde 101.

Par exemple, l'embout 106 peut contenir des diodes de type LED.

Le diamètre extérieur de la partie 105 de la membrane 104 en contact avec la face 107 du transducteur ultrasonore 103 est par exemple compris entre 3 et 25 mm.

La partie 105 de la membrane 104 en contact avec la face 107 du transducteur ultrasonore 103 a par exemple une épaisseur comprise entre 50 µm et 5 mm.

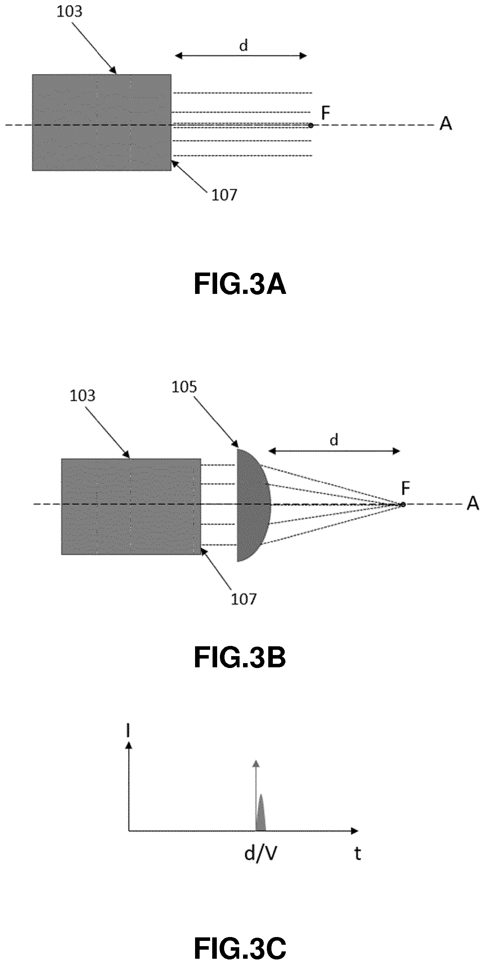

Comme illustré sur la figure 3B, la partie 105 de la membrane 104 peut constituer une lentille acoustique capable de focaliser le faisceau ultrasonore.

Par souci de clarté, la partie 105 de la membrane 104 a été représentée comme étant indépendante du transducteur ultrasonore 103 sur la figure 3B mais en réalité, la partie 105 de la membrane 104 est en contact avec la face 107 du transducteur ultrasonore 103.

Sur les figures 3A et 3B, le transducteur ultrasonore 103 émet un faisceau ultrasonore dont la direction de propagation est parallèle à l'axe A de symétrie du transducteur ultrasonore 103.

Sur la figure 3B, le faisceau ultrasonore est focalisé par la lentille acoustique en un point F situé à une distance d de la partie 105 de la membrane 104, constituant le foyer de la lentille acoustique. Tous les rayons parviennent au même moment au foyer F ce qui est favorable en termes de durée de la réponse impulsionnelle de diffraction du système.

Sur la figure 3A, en l'absence de la partie 105 de la membrane 104, le trajet du faisceau ultrasonore n'est pas dévié. Le foyer F est défini par la focalisation « naturelle » du transducteur ultrasonore 103 de type piston avec comme inconvénient que la durée de la réponse impulsionnelle de diffraction est plus large qu'en focalisant à l'aide d'une lentille acoustique.

Sur la figure 3C sont représentées les réponses impulsionnelles de diffraction au point F dans le cas où le faisceau ultrasonore n'est pas focalisé et dans le cas où le faisceau ultrasonore est focalisé au point F par la partie 105 de la membrane 104 constituant une lentille acoustique. V est la vitesse de propagation des ondes ultrasonores dans le milieu considéré.

On note que la réponse impulsionnelle de diffraction à des instants proches de l'instant égal à d/V est plus courte dans le cas d'une focalisation par lentille ce qui est plus favorable à l'obtention d'une bonne résolution axiale et latérale de la sonde 100.

L'intensité du signal réfléchi par le point F dans le cas de la présence d'une lentille acoustique est également plus importante que dans le cas de l'absence de lentille acoustique puisque les rayons sont sommés de manière cohérente au foyer F.

La réponse impulsionnelle de diffraction au point F dans le cas de l'absence de lentille acoustique est plus étalée temporellement que dans le cas de la présence d'une lentille acoustique car les rayons ultrasonores ne se somment pas de manière parfaitement cohérente du fait qu'ils parcourent des distances plus ou moins grandes pour atteindre le foyer F.

La lentille acoustique peut être concave ou convexe selon que la vitesse de propagation des ultrasons dans la lentille est inférieure ou supérieure à celle des ultrasons dans l'eau.

## Revendications

1. Sonde (100) d'élastographie transitoire comportant :
- Un corps de sonde (101) ;
- Un transducteur ultrasonore (103) configuré pour générer un faisceau ultrasonore selon un axe, le faisceau ultrasonore étant généré depuis une face (107) du transducteur ultrasonore (103) destinée à être en contact avec le corps d'un patient ;
- Un vibreur (102) situé à l'intérieur du corps de sonde (101) et agencé de manière à induire un mouvement du transducteur ultrasonore (103) selon un axe prédéfini ;
le transducteur ultrasonore (103) étant monté sur le vibreur (102) de manière que l'axe prédéfini et l'axe du faisceau ultrasonore soient confondus, la sonde (100) comportant en outre une membrane (104) d'étanchéité épousant les contours extérieurs du transducteur ultrasonore (103)
**caractérisé en ce que** ladite membrane (104) recouvre la face (107) du transducteur ultrasonore (103).

2. Sonde (100) selon la revendication 1, **caractérisée en ce que** l'ensemble transducteur ultrasonore (103) et membrane (104) constitue un embout détachable (106).

3. Sonde (100) selon l'une quelconque des revendications précédentes, **caractérisée en ce que** le transducteur ultrasonore (103) présente un axe de symétrie correspondant à l'axe du faisceau ultrasonore.

4. Sonde (100) selon l'une quelconque des revendications précédentes, **caractérisée en ce que** le transducteur ultrasonore (103) est relié au corps de sonde (101) par la membrane (104).

5. Sonde (100) selon l'une quelconque des revendications précédentes, **caractérisée en ce que** la membrane (104) est en élastomère.

6. Sonde (100) selon la revendication 5, **caractérisée en ce que** la membrane (104) est en élastomère de type silicone.

7. Sonde (100) selon l'une quelconque des revendications précédentes, **caractérisée en ce que** la partie (105) de la membrane (104) en contact avec la face (107) du transducteur ultrasonore (103) forme une lentille acoustique configurée pour focaliser le faisceau ultrasonore.

8. Sonde (100) selon la revendication 7, **caractérisée en ce que** la partie (105) de la membrane (104) en contact avec la face (107) du transducteur ultrasonore (103) est convexe.

9. Sonde (100) selon la revendication 7, **caractérisée en ce que** la partie (105) de la membrane (104) en contact avec la face (107) du transducteur ultrasonore (103) est concave.

10. Sonde (100) selon l'une quelconque des revendications précédentes, **caractérisée en ce que** la membrane (104) est réalisée dans un matériau électriquement isolant.

11. Sonde (100) selon l'une quelconque des revendications précédentes, **caractérisée en ce que** la membrane (104) est collée au transducteur ultrasonore (103).

12. Sonde (100) selon l'une quelconque des revendications précédentes, **caractérisée en ce que** la partie de la membrane (104) située entre le transducteur ultrasonore (103) et le corps de sonde (101) est déformable.

13. Sonde (100) selon l'une quelconque des revendications 7 à 9, **caractérisée en ce que** le diamètre extérieur de la partie (105) de la membrane (104) en contact avec la face (107) du transducteur ultrasonore (103) est compris entre 3 et 25 mm.

14. Sonde selon l'une quelconque des revendications 7 à 9 précédentes, **caractérisée en ce que** l'épaisseur de la partie (105) de la membrane (104) en contact avec la face (107) du transducteur ultrasonore (103) est comprise entre 50 µm et 5 mm.

15. Sonde (100) selon l'une quelconque des revendications précédentes, **caractérisée en ce que** tout ou partie du transducteur ultrasonore (103) a une forme de cône tronqué, la face (107) du transducteur ultrasonore (103) correspondant à la base du cône tronqué d'aire minimale.

## Patentansprüche

1. Sonde (100) für transiente Elastographien, umfassend:
Einen Sondenkörper (101);
Einen Ultraschallwandler (103), der so konfiguriert ist, dass er einen Ultraschallstrahl entlang einer Achse erzeugt, wobei der Ultraschallstrahl von einer Fläche (107) des Ultraschallwandlers (103) erzeugt wird, die dazu bestimmt ist, den Körper eines Patienten zu berühren;
Einen Vibrator (102), der sich im Inneren des Sondenkörpers (101) befindet und so angeordnet ist, dass er eine Bewegung des Ultraschallwandlers (103) entlang einer vordefinierten Achse induziert;
wobei der Ultraschallwandler (103) auf dem Vibrator (102) so montiert ist,
dass die vordefinierte Achse und die Achse des Ultraschallstrahls zusammenfallen, wobei die Sonde (100) außerdem eine Dichtungsmembran (104) umfasst, die sich an die Außenkonturen des Ultraschallwandlers (103) anpasst, **dadurch gekennzeichnet, dass** die Membran (104) die Fläche (107) des Ultraschallwandlers (103) bedeckt.

2. Sonde (100) nach Anspruch 1, **dadurch gekennzeichnet, dass** die Einheit aus Ultraschallwandler (103) und Membran (104) ein abnehmbares Anschlussstück (106) bildet.

3. Sonde (100) nach einem der vorhergehenden Ansprüche, **dadurch gekennzeichnet, dass** der Ultraschallwandler (103) eine Symmetrieachse aufweist, die der Achse des Ultraschallstrahls entspricht.

4. Sonde (100) nach einem der vorhergehenden Ansprüche, **dadurch gekennzeichnet, dass** der Ultraschallwandler (103) über die Membran (104) mit dem Sondenkörper (101) verbunden ist.

5. Sonde (100) nach einem der vorhergehenden Ansprüche, **dadurch gekennzeichnet, dass** die Membran (104) aus einem Elastomer besteht.

6. Sonde (100) nach Anspruch 5, **dadurch gekennzeichnet, dass** die Membran (104) aus einem Silikon-Elastomer besteht.

7. Sonde (100) nach einem der vorhergehenden Ansprüche, **dadurch gekennzeichnet, dass** der Teil (105) der Membran (104), der mit der Fläche (107) des Ultraschallwandlers (103) in Kontakt ist, eine akustische Linse bildet, die so gestaltet ist, dass sie den Ultraschallstrahl fokussiert.

8. Sonde (100) nach Anspruch 7, **dadurch gekennzeichnet, dass** der Teil (105) der Membran (104), der mit der Fläche (107) des Ultraschallwandlers (103) in Kontakt ist, konvex ist.

9. Sonde (100) nach Anspruch 7, **dadurch gekennzeichnet, dass** der Teil (105) der Membran (104), der mit der Fläche (107) des Ultraschallwandlers (103) in Kontakt ist, konkav ist.

10. Sonde (100) nach einem der vorhergehenden Ansprüche, **dadurch gekennzeichnet, dass** die Membran (104) aus einem elektrisch isolierenden Material hergestellt ist.

11. Sonde (100) nach einem der vorhergehenden Ansprüche, **dadurch gekennzeichnet, dass** die Membran (104) an den Ultraschallwandler (103) angeklebt ist.

12. Sonde (100) nach einem der vorhergehenden Ansprüche, **dadurch gekennzeichnet, dass** der Teil der Membran (104), der sich zwischen dem Ultraschallwandler (103) und dem Sondenkörper (101) befindet, verformbar ist.

13. Sonde (100) nach einem der Ansprüche 7 bis 9, **dadurch gekennzeichnet, dass** der Außendurchmesser des Teils (105) der Membran (104), der mit der Fläche (107) des Ultraschallwandlers (103) in Kontakt ist, zwischen 3 und 25 mm beträgt.

14. Sonde nach einem der vorhergehenden Ansprüche 7 bis 9, **dadurch gekennzeichnet, dass** die Dicke des Teils (105) der Membran (104), der mit der Fläche (107) des Ultraschallwandlers (103) in Kontakt ist, zwischen 50 µm und 5 mm beträgt.

15. Sonde (100) nach einem der vorhergehenden Ansprüche, **dadurch gekennzeichnet, dass** der gesamte oder ein Teil des Ultraschallwandlers (103) die Form eines Kegelstumpfes hat, wobei die Fläche (107) des Ultraschallwandlers (103) der Basis des Kegelstumpfes mit minimaler Fläche entspricht.

## Claims

1. A transient elastography probe (100) including:
- a probe body (101);
- an ultrasonic transducer (103) configured to generate an ultrasonic beam along an axis, the ultrasonic beam being generated from a face (107) of the ultrasonic transducer (103) intended to be in contact with the body of a patient;
- a vibrator (102) located inside the probe body (101) and arranged to induce movement of the ultrasonic transducer (103) along a predefined axis;
the ultrasonic transducer (103) being mounted to the vibrator (102) so that the predefined axis and the axis of the ultrasonic beam are coincident,
the probe (100) further including a sealing membrane (104) snugly fitting the external contours of the ultrasonic transducer (103), **characterised in that** said membrane (104) covers the face (107) of the ultrasonic transducer (103).

2. The probe (100) according to claim 1, **characterised in that** the ultrasonic transducer (103) and membrane (104) assembly constitutes a detachable tip (106).

3. The probe (100) according to any of the preceding claims, **characterised in that** the ultrasonic transducer (103) has an axis of symmetry corresponding to the axis of the ultrasonic beam.

4. The probe (100) according to any of the preceding claims, **characterised in that** the ultrasonic transducer (103) is connected to the probe body (101) through the membrane (104).

5. The probe (100) according to any of the preceding claims, **characterised in that** the membrane (104) is of elastomer.

6. The probe (100) according to claim 5, **characterised in that** the membrane (104) is of silicone type elastomer.

7. The probe (100) according to any of the preceding claims, **characterised in that** the part (105) of the membrane (104) in contact with the face (107) of the ultrasonic transducer (103) forms an acoustic lens configured to focus the ultrasonic beam.

8. The probe (100) according to claim 7, **characterised in that** the part (105) of the membrane (104) in contact with the face (107) of the ultrasonic transducer (103) is convex.

9. The probe (100) according to claim 7, **characterised in that** the part (105) of the membrane (104) in contact with the face (107) of the ultrasonic transducer (103) is concave.

10. The probe (100) according to any of the preceding claims, **characterised in that** the membrane (104) is made of an electrically insulating material.

11. The probe (100) according to any of the preceding claims, **characterised in that** the membrane (104) is bonded to the ultrasonic transducer (103).

12. The probe (100) according to any of the preceding claims, **characterised in that** the part of the membrane (104) located between the ultrasonic transducer (103) and the probe body (101) is deformable.

13. The probe (100) according to any of claims 7 to 9, **characterised in that** the external diameter of the part (105) of the membrane (104) in contact with the face (107) of the ultrasonic transducer (103) is between 3 and 25 mm.

14. The probe according to any of the preceding claims 7 to 9, **characterised in that** the thickness of the part (105) of the membrane (104) in contact with the face (107) of the ultrasonic transducer (103) is between 50 µm and 5 mm.

15. The probe (100) according to any of the preceding claims, **characterised in that** all or part of the ultrasonic transducer (103) is of a truncated cone shape, the face (107) of the ultrasonic transducer (103) corresponding to the base of the truncated cone with a minimum area.
